# EUROPEAN PATENT APPLICATION

(11) **EP 1 914 317 A1**
(43) Date of publication of application: **23.04.2008**
(21) Application number: 06022097.7
(22) Date of filing: 21.10.2006
(51) Int. Cl.: C12Q 1/68

(54) **A method for qualitative and quantitative detection of short nucleic acid sequences of about 8 to 50 nucleotides in length**

(71) Applicant: Biolytix AG, 4108 Witterswil (CH)
(72) Inventor: Moor, Dominik, 5000 Aarau (CH); Seyfarth, Ralf, 4102 Binningen (CH); Brodmann, Peter, 4410 Liestal (CH)

(57) **Abstract**

The present invention concerns a new analytical method for qualitative and quantitative detection of short nucleic acid sequences, such as oligonucleotides or fragmented nucleic acid sequences of about 8-50 nucleotides in length. The invention relates to the introduction of modified nucleic acids into an oligonucleotide probe that hybridizes to the target sequence such that amplification and quantitation of the short nucleic acid sequence is enabled and sensitivity and specificity of the reaction is increased. The invention also embraces test kits for performing nucleic acid amplification to detect and quantitate short nucleic acid sequences and processes for preparing such and the use of new analytical methods.

SEQUENCE LISTING

<110> Biolytix AG
      Moor, Dominik
      Brodmann, Peter
      Seyfarth, Ralf

<120> A new method for qualitative and quantitative detection of short
      nucleic acid sequences of about 8-50 nucleotides in length

<130> Biolytix 102

<140> 06022097.7-1222
<141> 2007-01-03

<160> 5

<170> PatentIn version 3.3

<210> 1
<211> 18
<212> DNA
<213> Artificial

<220>
<223> Antisense Phosphorothioate Oligodeoxyribonucleotide to Homo
      sapiens B-cell CLL/lymphoma 2 (BCL2) mRNA


<220>
<221> modified_base
<222> (1)..(18)

<300>
<308> GenBank / CS080249
<309> 2005-05-09
<313> (1)..(18)

<300>
<302> Method for identifying interferon mimics
<310> WO2005038052
<312> 2005-04-28
<313> (1)..(18)

<400> 1
tctcccagcg tgcgccat                                                    18


<210> 2
<211> 38
<212> DNA
<213> Artificial

<220>
<223> capture probe contains LNA (Locked Nucleic Acid) bases at
      postitions: 30, 32, 34, 36, 37


<220>
<221> modified_base
<222> (30)..(37)

<400> 2

tttggagcct gggacgtgct ggatacgaca tggcgcac                                38

<210>  3
<211>  17
<212>  DNA
<213>  Artificial

<220>
<223>  forward primer

<220>
<221>  primer_bind
<222>  (1)..(17)

<400>  3
ccgttctccc agcgtgc                                                      17

<210>  4
<211>  18
<212>  DNA
<213>  Artificial

<220>
<223>  reverse primer

<220>
<221>  primer_bind
<222>  (1)..(18)

<400>  4
tttggagcct gggacgtg                                                     18

<210>  5
<211>  18
<212>  DNA
<213>  Artificial

<220>
<223>  TaqMan MGB probe

<220>
<221>  primer_bind
<222>  (1)..(18)

<400>  5
tggatacgac atggcgca                                                     18

## Description

The new invented analytical methods and test kits qualitatively and quantitatively detect short oligomeric nucleic acids, such as antisense oligonucleotides, phosphorothioate oligonucleotides and phosphodiester oligonucleotides, in blood serum, tissue samples and other matrices.

The method has a detection limit (LOD) of about 50 fM (0.3 pg/ml for antisense phosphorothioate oligonucleotide G3139 and also for the phosphodiester analog), which corresponds to an absolute amount of 0.75 attomole of target oligonucleotides in 15 µl sample volume. The limit of quantitation (LOQ) is 100 fM and the method has a broad dynamic range of accurate quantitation of about 7 log-values (100 fM - 0.5 µM). These method characteristics are superior to all known methods applied for short nucleic acid quantitation.

The major advantages of the present invention over other published oligonucleotide quantitation methods are the increased sensitivity, the high specificity, the good discrimination, the high accuracy and precision, the good reproducibility and robustness, the broad dynamic range, the low sample requirements, the lack of laborious sample clean-up procedures or sample derivatization steps, the fast and easy sample processing, and the high-throughput capability. The method is based on a real-time PCR approach, which is state-of-the-art for nucleic acid quantitation.

Those methods being new and inventive will replace conventional analytical methods like mass spectrometry (MS), capillary gel electrophoresis (CGE), high performance liquid chromatography (HPLC) and hybridization enzyme-linked immunosorbent assays (ELISA).

### State of the Art

The quantitative detection of short double-stranded or single-stranded oligomeric nucleic acids including antisense oligonucleotides, short interfering RNA (siRNA) and microRNA (miRNA) in cells, blood plasma and tissues becomes increasingly important. A special interest has grown in antisense oligonucleotides as pharmacological tools and therapeutic agents. Different techniques and methods have been developed in the past for the quantitation of short oligonucleotides, to study their therapeutic use, their stability in biological fluids and target specificity. A number of methods to detect short nucleic acid sequences are cited in the literature and disclosed in published patent applications.

The major advantages of the present invention over other oligonucleotide quantitation methods are the increased sensitivity, the high specificity, the high accuracy and precision, the broad dynamic range, the fast and easy sample processing and the high-throughput capability.
In the following published oligonucleotide quantitation methods are described and compared to the present invention.

### Hybridisation assays:

In nucleic acid hybridization assays an oligonucleotide sequence complementary to the target oligonucleotide is covalently bound to a solid phase and either a sandwich hybridzation assay or competitive assay is performed for target sequence determination with a labeled tracer oligonucleotide.

A method for quantitation of phosphorothioate oligonucleotides in biological fluids and tissues is described by Temsamani et al., Anal Biochem. 1993. 215(1), p.54 - 58, in which the target antisense oligonucleotide is immobilized on a nylon membrane and a complementary tracer oligonucleotide is used to quantitate the fixed analyte. The described method uses an inconvenient solvent extraction procedure and radiolabeled tracer oligonucleotides.

Overhoff et al., Nucleic Acids Research 2004. 32(21), p.e170, describe the quantitation of siRNA using the corresponding ³²P-labeled sense strand of siRNA. After liquid hybridization of siRNA and labeled probe the unbound probe is removed with an RNase digest and the samples are analyzed by polyacrylamide gel electrophoresis followed by blotting onto nylon membrane and quantification. The disadvantages of this method are the use of radiolabeled oligonucleotides and the laborious extraction and purification procedure.

### Enzyme linked immunosorbent assay (ELISA), competitive, non-competitive, sandwich:

Deverre et al., Nucleic Acids Research 1997. 25(18), p.3584-3589, developed an enzyme competitive hybridization assay for determination of mouse plasma concentrations of a 15mer antisense phosphodiester oligodeoxyribonucleotide and phosphorothioate analogs. The principle of this assay involves competitive hybridization of a biotinylated tracer oligonucleotide and the target antisense oligonucleotide to the solid-phase immobilized sense-oligonucleotide that is covalently linked to the surface of polystyrene microwells. The tracer oligonucleotide is then assayed after reaction with a streptavidin-acetylcholinesterase conjugate using a colorimetric detection method. The limit of quantitation of this method was 900 pM, which is 9'000 fold less sensitive than our invented method (LOQ = 100 fM).

A competitive hybridization assay is described by Boutet et al., Biochem Biophys Res Commun. 2000. 268(1), p.92-98, that quantifies phosphorothioate and phosphodiester oligonucleotides in biological fluids without extraction, by the use of two different probes and a fluorescent transfer process. The sensitivity of the assay for phosphorothioate and phosphodiester oligonucleotides in plasma was 800 pM and 200 pM, respectively. The limit of quantitation of our invention is 100 fM for both phosphorothioate and phosphodiester oligonucleotides, which is an increase in sensitivity of 8'000 fold and 2'000 fold, respectively.

Yu et al., Analytical Biochemistry 2002. 304(1), p.19-25, describe a non-competitive hybridization-ligation heterogeneous enzyme-linked immunosorbent assay for the quantitation of antisense phosphorothioate oligodeoxynucleotides in human plasma. The principle of this assay is based on heterogeneous non-competitive binding of the antisense target oligonucleotide to an immobilized probe oligonucleotide, followed by ligation of a fluorescent signal probe. Detection and quantitation is performed using a fluorescence microtiter plate reader. The limit of quantitation of the method in human plasma was 50 pM, which is 500 fold less sensitive than our invention (LOQ = 100 fM).

Further the linear range of the method was 0.05 nM to 2 nM (about 2 log-values), whereas our invention has a linear range of 100 fM to 0.5 µM (about 7 log-values).

A hybridization-based enzyme-linked immunosorbent assay method for quantification of phosphorothioate oligonucleotides in biological fluids (plasma and cellular matrices) is described by Wei et al., Pharm Res. 2006. 23(6), p.1251-1264. The method is based on hybridization of the phosphorothioate target to a biotin-labeled capture probe, followed by ligation with digoxigenin-labeled detection probe. The bound duplex is then detected by anti-digoxigenin-alkaline phosphatase conjugate using a colorimetric detection method. The limit of quantitation of this assay was 50 pM and the linear range from 0.05 nM to 10 nM (about 2 log-values), whereas our invented method has a linear range of 100 fM to 0.5 µM (about 7 log-values) and has a 500 fold higher sensitivity (LOQ of our invention = 100 fM).

The use of oligonucleotide probes containing locked nucleic acids (LNA) to increase sensitivity and specificity of a colorimetric hybridization assay is described by Efler et al., Oligonucleotides 2005, 15(2), p.119-131. The limit of detection for this assay was 2.8 pg/ml or 40 attomoles of target oligonucleotides, and the linear range was 7.8 - 1000 pg/ml (about 2 log-values). Our invented method has a 55 fold higher sensitivity with a detection limit of 0.75 attomoles, and has a broader linear detection range of about 7 log-values (0.6 pg/ml - 3 µg/ml). Further the plasma sample requirements of this method (and most other ELISA-based methods) was 100µl, whereas our invented method has a sample requirement of only 15µl.

### Capillary gel electrophoresis/UV-detection:

Capillary gel electrophoresis (CGE) is a well-established technique for quantitation of short nucleic acid sequences and has been used as the mayor bioanalytical method in many clinical trials. CGE allows the separation of parent compound from chain-shortened metabolites with good resolution. Following CGE separation an UV-detection at 260 nm is most frequently applied, which has a LOD value of 70 ng/ml in plasma. This sensitivity is sufficient to monitor pharmacokinetic behaviour but is insufficient to characterize the terminal elimination phase of the oligonucleotides in plasma. This requirement is met by our quantitation method, which has a 100'000 fold higher sensitivity of 0.3 pg/ml in plasma. Further our invention does not need extensive extraction methods and inconvenient sample clean-up procedures or on-column derivatization steps to improve sensitivity, as described by Shang et al., Acta Pharmcol Sin. 2004. 25(6), p.801-806, and Yu et al., Drug Discovery & Development 2004. 7(2), p.195-203.

### Mass spectrometry:

Another well-established technique for quantitation of short nucleic acid sequences is mass spectrometry (MS). Different methods for quantitation of e.g. antisense oligonucleotides and their metabolites are described. Liquid chormatography prior MS and tandem MS/MS methods facilitates quantitation of oligonucleotides in plasma samples but still a solid-phase extraction procedure is necessary for antisense oligonucleotide detection.
Yu et al., Drug Discovery & Development 2004. 7(2), p.195-203, describe a MS method for plasma samples, for which the dynamic range was between 1 and 2000 ng/ml and the LOD was 100 pg on the column, which is equivalent to 5 ng/ml in plasma. Compared to the MS methods our invention has a 10'000 fold higher sensitivity (LOD = 0.0045 pg, equivalent to 0.3 pg/ml in plasma sample) and a larger dynamic range of accurate quantitation of about 7 log-values (100 fM - 0.5 µM).
Dai et al., J. Chromatotogr. B. 2005. 825(2), p.201-213, describe a HPLC-MS/MS quantification method for a phosphorothioate antisense oligonucleotide in human and rat plasma with a LOQ of 17.6 nM. The LOQ of our invention is 100 fM, which corresponds to a 100'000 fold increased sensitivity.
A tandem light chromatography-UV detection-MS method is described by Gilar et al., Oligonucleotides 2003. 13(4), p.229-243, which has an estimated LOQ of < 1 picomole of oligonucleotide injected on-column. The LOQ of our invented method is 1.5 attomole, which is > 5 orders of magnitude more sensitive.

### Alternative nucleic acid quantitation methods:

The further mentioned nucleic acid quantitation methods have not been shown to work for the quantitative analysis of oligonucleotides in blood plasma and other biological samples and/or are less sensitive and/or less specific compared to our described method.

### Electroactive hybridisation probes:

An alternative technology for oligonucleotide quantitation is described by Jenkins et al., Anal Chem. 78(7), p.2314-2318, by which mixed monolayers of electroactive hybridization probes on gold surfaces of a disposable electrode are used. Hybridization of the target sequence to the ferrocene-labeled hairpin probes diminishes cyclic redox currents, presumably due to a displacement of the label from the electrode. Detection limits were demonstrated down to nearly 100 fM, but this technique has not been shown to work for the analysis of oligonucleotides in blood plasma samples and is not used as a standard quantitative bioanalytical method so far.

### Ligation assay:

A ligation assay described by Dille et al in Journal of Clinical Microbiology, 1993, 31(3), p. 720-731 shows an amplification of *Clamydia trachomatis* DNA by polymerase chain reaction which was compared with amplification by ligase chain reaction (LCR). Both amplification procedures were able to consistently amplify amounts of DNA equivalent to three *C.trachomatis* elementary bodies. All 15 *C.trachomatis* serovars were amplified to detectable levels by LCR ,and no DNA fom 16 organisms potentially found in clinical specimen or from *Chlamydia psittaci* and *Chlamidia pneumoniae* was amplyfied by LCR.

### Deoxyribozyme assay:

A binary deoxyribozyme ligase was engineered by Tabor et al., Nucleic Acids Research 2006. 34(8), p.2166-2177, of which the half-deoxyribozymes can be activated by a bridging oligonucleotide to carry out a ligation reaction. The engineered deoxyribozyme can recode nucleic acid information by "reading" one sequence through hybridization and then "writing" a separate sequence by ligation, which can then be used as template for amplification by PCR. This technique has not been shown to work for the quantitative analysis of oligonucleotides in blood plasma samples and is not used as a standard quantitative bioanalytical method so far.

### DNA binding dyes:

Gray et al., Antisense Nucleic Acid Drug Dev. 1997. 7(3) p.133-140, described the use of a single-stranded DNA binding fluorophore, OliGreen, that allowed quantitation of oligonucleotides and analogs in calf, mouse, and human plasma samples. The linear range of the method was reported to be 15 - 500 nM. Our invented method has a 150'000 fold increased sensitivity and a broader dynamic range of about 7 log-values (100 fM - 0.5µM).

### PCR-based assays:

A PCR-based quantitative analysis method of microRNAs and short-interfering RNAs is described by Raymond et al., RNA 2005. 11(11) p.1737-1744. The method relies on primer extension conversion of RNA to cDNA by reverse transcription followed by quantitative real-time PCR. LNA bases in the PCR reverse primer increased the performance of the assay. The assay allows measurements in the femtomolar range and has a high dynamic range of 6-7 orders of magnitude, which is comparable to our invented method. This method is designed for quantitation of short RNA molecules and does not allow for quantitation of antisense phosphorothioate oligonucleotides in blood plasma samples.

### Stem-loop RT-PCR:

The real-time quantification of microRNAs and other small RNAs by stem-loop RT-PCR is described by Chen et al., Nucleic Acids Research 2005. 33(20) p.1-9. For triggering the reverse transcription of RNA into cDNA the method uses RT primers that form a stem-loop structure and show better specificity and sensitivity than linear ones. Quantitation of cDNA is then done using real-time PCR assays that exhibit a dynamic range of seven orders of magnitude, which is comparable to our invention. This PCR-based method is designed for the quantitation of small RNA molecules and cannot be used for quantitation of antisense phosphorothioate oligonucleotides in blood plasma samples.

### Isothermal amplification:

Tan et al., Anal Chem. 2005. 77(24) p.7984-7992, describe an isothermal nucleic acid amplification reaction that detect short DNA sequences. The method is combined with visual, colorimetric readout based on aggregation of DNA-functionalized gold nanospheres. The reaction is initiated by the trigger oligonucleotide which is exponentially amplified and converted to a universal reporter oligonucleotide capable of bridging two sets of DNA-functionalized gold colloids. The method permits detection of 100 fM trigger oligonucleotide in 10 min, but this technique has not been shown to work for the analysis of e.g. phosphorothioate oligonucleotides in blood plasma samples and is not used as a standard quantitative bioanalytical method so far.

### Description of the invention:

Disclosed is a method of qualitative and quantitative detecting a short nucleic acid sequence of interest in a sample, the method comprising contacting the sample with a capture probe; the capture probe comprising a portion complementary to part of the sequence of interest and so capable of hybridising thereto, and a portion non-complementary to the sequence of interest; causing extension of the sequence of interest with a nucleic acid polymerase, using the capture probe as a template; causing extension of the capture probe with a nucleic acid polymerase, using the sequence of interest as a template; and qualitative and quantitative detecting directly or indirectly the extended sequence of interest and the extended capture probe using a nucleic acid amplification reaction, so as to indicate the presence and amount of the sequence of interest; characterized in that the primers used for nucleic acid amplification comprising a portion complementary to the extension of the sequence of interest and of the extension of the capture probe, thereby preventing nucleic acid amplification in the absence of the sequence of interest.
The method has a detection limit of 50 fM (0.3 pg/ml), which corresponds to 0.75 attomoles of target molecules and has a dynamic range of about 7 log-values.

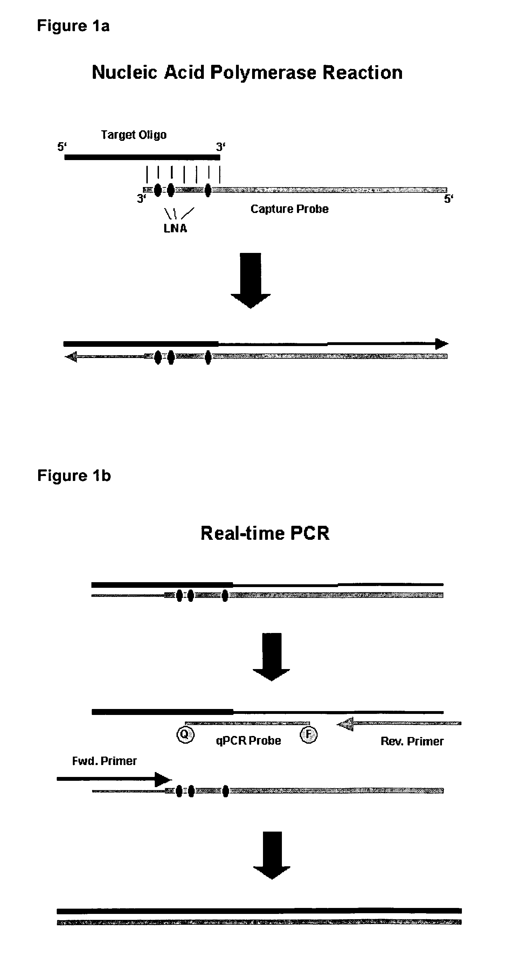

Figures 1a-1b show a schematic drawing of the invented method comprising (a) a nucleic acid polymerase reaction for target/probe extension and (b) a real-time PCR assay for quantitative analysis of target molecules.

In preferred embodiments the present invention also fulfils all the aforementioned desiderata. This may be achieved through the hybridisation of an oligonucleotide probe that contains complementary target specific regions, such that in the presence of the target sequence of interest, the probe hybridizes to the complementary target sequence.

In a first aspect the invention provides a capture probe for use in a method of qualitative and quantitative detecting a short nucleic acid target sequence of interest, comprising a portion complementary to part of the sequence of interest and so capable of hybridizing thereto, and a portion non-complementary to the sequence of interest, both unpaired ends of target sequence and hybridized capture probe serving as templates for extension with a nucleic acid polymerase.

The target strand may comprise any nucleic acid and/or nucleic acid analogs (DNA; RNA, LNA, PNA, PTO, MGB, 2'-MOE) sequence of interest, such as an antisense oligonucleotide, a siRNA, a miRNA, a strongly fragmented DNA (such that the method may be used to detect and quantify the presence of a species-specific sequence in a sample), or any other short nucleic acid single-stranded or double-stranded sequence of about 8-50 nucleotides in length.

The hybridisation of the capture probe to the sequence of interest forms a nucleic acid duplex of complementary sequences, having both unpaired ends of non-complementary sequences. The capture probe preferably comprise DNA, LNA (locked nucleic acid) or PNA (peptide nucleic acid), but may comprise RNA, MGB (minor groove binder), PTO (phosphorothioate oligonucleotide), 2'-MOE (2'-methoxyethyl) oligonucleotide, other nucleic acid analogs or any combination thereof.

LNA is a synthetic nucleic acid analogue, incorporating "internally bridged" nucleoside analogues. Synthesis of LNA, and properties thereof, have been described by a number of authors: Nielsen et al, (1997 J. Chem. Soc. Perkin Trans. 1, 3423); Koshkin et al, (1998 Tetrahedron Letters 39, 4381); Singh & Wengel (1998 Chem. Commun. 1247); and Singh et al, (1998 Chem. Commun. 455). LNA exhibits greater thermal stability when paired with DNA, than do conventional DNA/DNA heteroduplexes. However, LNA can be synthesised on conventional nucleic acid synthesising machines, whereas PNA cannot; special linkers are required to join PNA to DNA, when forming a single stranded PNA/DNA chimera. In contrast, LNA can simply be joined to DNA molecules by conventional techniques. Therefore, in some respects, LNA is to be preferred over PNA, for use in probes in accordance with the present invention.
In particular, the target specific region of the capture probe may comprise LNA and/or other nucleic acid analogs and the region non-complementary to the sequence of interest comprise DNA.

A number of nucleic acid amplification processes are cited in the literature and disclosed in published European and PCT patent applications. One such process known as polymerase chain reaction (PCR) is disclosed in U.S. Pat. No. 4,683,202 and has been well introduced wordwide.
The invention of the PCR has greatly improved the sensitivity and specificity of nucleic acid detection methods. PCR is a process for amplifying nucleic acids and involves the use of two nucleic acid primers (oligonucleotides), an agent for polymerization (e.g. thermostable DNA polymerase), a target nucleic acid template, nucleoside triphosphates, and successive cycles of denaturation of nucleic acid and annealing and extension of the primers to produce a large number of copies of a particular nucleic acid segment. With this method, segments of single copy genomic DNA can be amplified more than 10 million fold with very high specificity and fidelity.

Methods for detecting PCR products are particularly described in U.S. Pat. No. 4,683,195. Those methods require an oligonucleotide probe capable of hybridizing with the amplified target nucleic acid.

A number of agents have been described for labeling nucleic acids for facilitating detection of target nucleic acid. Suitable labels may provide signals detectable by fluorescence, radioactivity, colorimetry, X-ray diffraction or absorption, magnetism or enzymatic activity and include, for example, fluorophores, chromophores, radioactive isotopes, electron-dense reagents, enzymes, and ligands having specific binding partners.

U.S. Pat. No. 5,210,015 describes an alterative assay method for detecting amplified nucleic acids. The process employs the 5' to 3' nuclease activity of a nucleic acid polymerase to cleave annealed, labeled oligonucleotides from hybridized duplexes and release labeled oligonucleotide fragments for detection. The method is suitable for a quantitative detection of PCR products and requires a primer pair and a labeled oligonucleotide probe having a blocked 3'-OH terminus to prevent extension by the polymerase.

For the detection of short nucleic acid sequences the PCR method has its drawback in the limitation on the length of template nucleic acid sequence that is required for amplification. The minimal length is determined by the length of the primer and probe annealing sequences which should not overlap, and the sequence in-between. Therefore a template nucleic acid sequence of at least 50 basepairs is required and therefore not useable for less than 50 basepairs.

The present invention addresses and solves the needs for a PCR-based method that allows the qualitative and quantitative detection of short nucleic acid sequences (e.g. oligonucleotides) about between 8 to 50 nucleotides in length.

### Examples

### Example 1: Linear detection range of the invented method

To determine the linear range of accurate quantitation using our invented method the antisense phosphorothioate oligonucleotide G3139 (5'-3' sequence: TCT CCC AGC GTG CGC CAT) was selected as target molecule. A 10-fold serial dilution of PTO was prepared using a pipetting robot (CAS1200, Corbett Research). The dilution series consisted of 10 concentration levels with highest oligo-concentration of 2 µM.
The nucleic acid polymerase reaction was performed using the Klenow enzyme (Klenow fragment of *E.coli* DNA polymerase I). Of each PTO concentration level 15 µl were mixed with 5 µl of Klenow mastermix. The Klenow reaction consited of (final concentrations): 0.5 µM capture probe (5'-3' sequence: TTT GGA GCC TGG GAC GTG CTG GAT ACG ACA TGG CGC AC; bold letters = locked nucleic acid bases; Sigma-Proligo), 50 µM dNTP mix, 1x Klenow reaction buffer (Fermentas), 5 units of Klenow enzyme (Fermentas), 10 ng/µl human DNA, and H₂O to a final reaction volume of 20 µl. The concentration of G3139 antisense PTO in the reaction was 0.5 µM - 0.5 fM. In addition a non-template control without PTO was prepared. The Klenow reactions were incubated at 37°C for 20 minutes using a thermocycler.
Quantitation of target molecules in the Klenow reactions was done using real-time PCR. 5 µl of each 1:10 diluted Klenow reaction were mixed with 15 µl of PCR mastermix. The PCR reactions consisted of (final concentrations): 0.5 µM forward primer (5'-3' sequence: CCG TTC TCC CAG CGT GC), 0.5 µM reverse primer (5'-3' sequence: TTT GGA GCC TGG GAC GTG), 0.2 µM probe (5'-3' sequence: FAM-TGG ATA CGA CAT GGC GCA-MGB; Applied Biosystems), 1x qPCR MasterMix (Eurogentec) and H₂O to a final reaction volume of 20 µl. Real-time PCR was performed in an ABI 7900HT real-time PCR thermocycler (Applied Biosystems) using the following program: 2 min at 50°C, 10 min at 95°C, then 50 cycles of 15 sec at 95°C, 60 sec at 60°C.

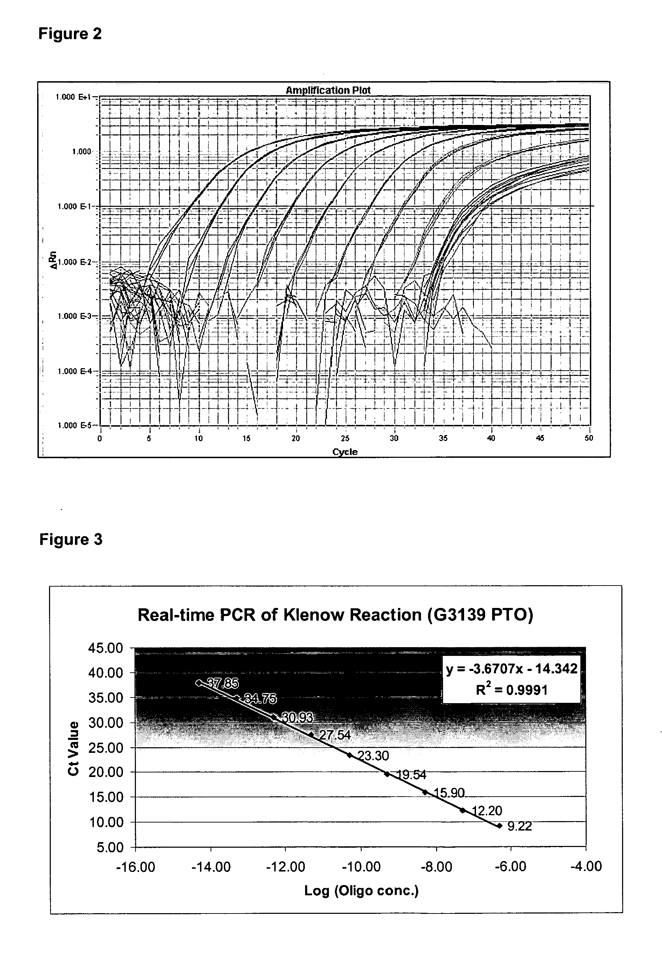

Figure 2 shows the PCR amplification plot of the nucleic acid polymerase reaction using 10-fold serially diluted G3139 antisense phosphorothioate oligonucleotide as template.
Figure 3 shows the linear regression analysis of the real-time PCR

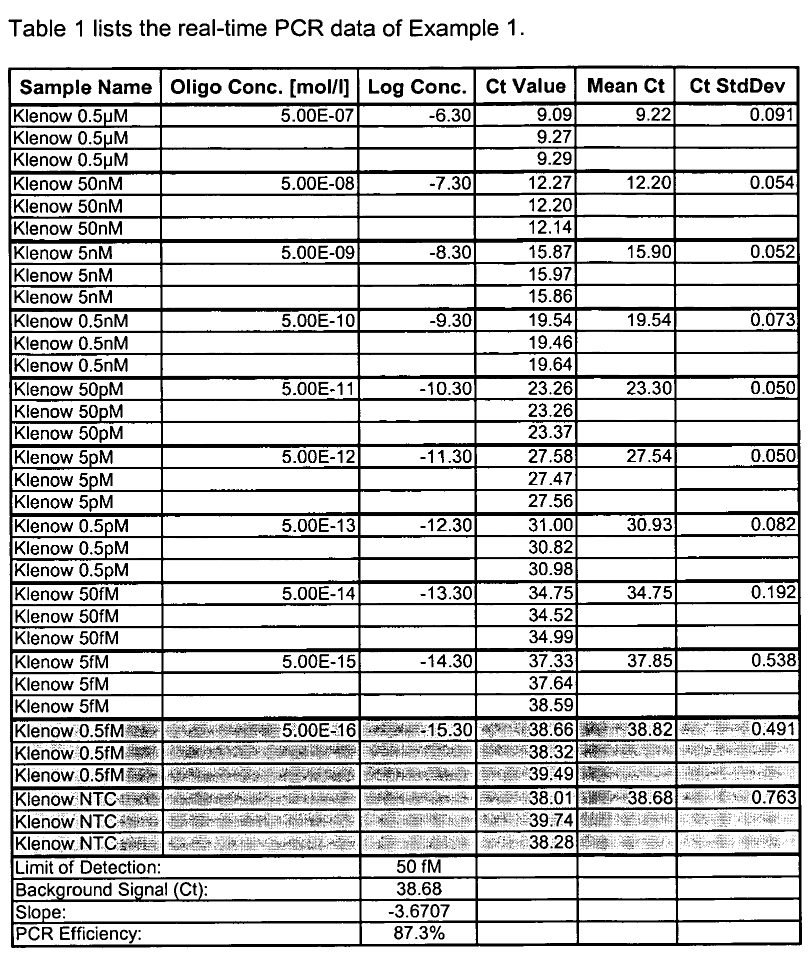

### Example 2: Limit of detection in plasma sample

To determine the method's detection limit (LOD) for antisense phosphorothioate oligonucleotide G3139 in plasma, human blood plasma was spiked with PTO to final concentrations of 50 pM - 5 fM in decade steps.
Of each concentration level 15 µl were mixed with 5 µl of Klenow mastermix, which was prepared as described in Example 1 but without adding human DNA (see Example 1). In addition a non-template control of plasma without PTO was prepared. The Klenow reactions were incubated at 37°C for 20 minutes using a thermocycler.

The Klenow reactions were purified using a NucleoSpin Extract II (Macherey-Nagel) DNA purification kit. The extraction was done according to the manufacturer's protocol. DNA was eluted in 100 µl elution buffer.
For quantitation of target molecules using real-time PCR 5 µl of each eluate were mixed with 15 µl of PCR mastermix (see Example 1). Real-time PCR was performed in a Rotorgene real-time PCR thermocycler (Corbett Research) using the following program: 2 min at 50°C, 10 min at 95°C, then 50 cycles of 15 sec at 95°C, 60 sec at 60°C.

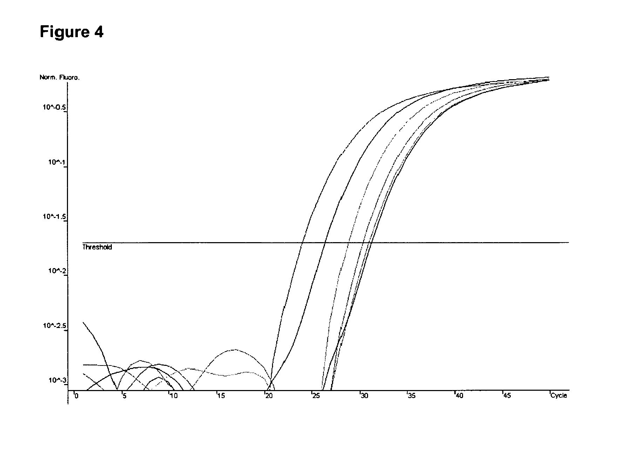

Figure 4 shows the PCR amplification plot of the nucleic acid polymerase reaction using human plasma spiked with G3139 antisense phosphorothioate oligonucleotide as template.

**Table 2 lists the real-time PCR data of Example 2.**

| **Conc. of G3139 PTO in Plasma** | **Ct Value** |
|---|---|
| 50 pM | 24.09 |
| 5 pM | 26.54 |
| 0.5 pM | 28.85 |
| 50 fM (Limit of detection) | 30.38 |
| 5 fM | 31.07 |
| NTC (non-template control) | 31.33 |

## Claims

1. A method of qualitative and quantitative detecting a short nucleic acid sequence of interest in a sample, the method comprising (**a**) contacting the sample with a capture probe; wherein the capture probe comprises a portion complementary to part of the sequence of interest and so capable of hybridising thereto, and a portion non-complementary to the sequence of interest; (**b**) causing extension of the sequence of interest with a nucleic acid polymerase, using the capture probe as a template; causing extension of the capture probe with a nucleic acid polymerase, using the sequence of interest as a template; and (**c**) qualitative and quantitative detecting directly or indirectly the extended sequence of interest and the extended capture probe using a nucleic acid amplification reaction, so as to indicate the presence and amount of the sequence of interest; **characterised in that** the primers used for nucleic acid amplification comprising a portion complementary to the extension of the sequence of interest and of the extension of the capture probe, thereby preventing nucleic acid amplification in the absence of the sequence of interest.

2. A method according to claim 1, wherein the sequence of interest is about between 8-50 nucleotides in length.

3. A method according to claim 1, wherein the sequence of interest comprise DNA, RNA, PTO (phosphorothioate oligonucleotide), 2'-MOE (2'-methoxyethyl) oligonucleotide, LNA (locked nucleic acid), MGB (minor groove binder), PNA (peptide nucleic acid), or any combination thereof.

4. A method according to claim 1, wherein the sequence of interest is a single-stranded molecule, a double-stranded molecule or any combination thereof.

5. A method according to claim 1, wherein the capture probe comprise DNA, PTO (phosphorothioate oligonucleotide), 2'-MOE (2'-methoxyethyl) oligonucleotide, RNA, LNA (locked nucleic acid), MGB (minor groove binder), PNA (peptide nucleic acid) or any combination thereof.

6. A method according to claim 1, wherein the sequence of the capture probe is such that hybridisiation to the sequence of interest forms an unpaired end of the capture probe.

7. A method according to claim 1, wherein the sequence of the capture probe is such that hybridisiation to the sequence of interest forms an unpaired end of the sequence of interest.

8. A method according to claim 1, wherein the sequence of interest is extended with a nucleic acid polymerase in an isothermal or in a thermal reaction using the unpaired end of the capture probe as template.

9. A method according to claim 1, wherein the capture probe is extended with a nucleic acid polymerase in an isothermal or in a thermal reaction using the unpaired end of the sequence of interest as template.

10. A method according to claim 1, wherein extension of the sequence of interest and extension of the capture probe results in formation of sequences suitable for primer annealing.

11. A method according to claim 1, wherein extension of the sequence of interest and/or extension of the capture probe results in formation of nucleic acid sequences having ribozyme activity (ligase-, nuclease-, or any other catalytic activity).

12. A method according to claim 10, wherein the formed sequences for primer annealing serve for isothermal or thermal nucleic acid amplification of the extended sequence of interest and the extended capture probe.

13. A method according to claim 12, wherein the amplification process serves for direct or indirect detection of the synthesized nucleic acid sequences.

14. A method according to claim 12, wherein the primers used for nucleic acid amplification comprise DNA, PTO (phosphorothioate oligonucleotide), 2'-MOE (2'-methoxyethyl) oligonucleotide, RNA, LNA (locked nucleic acid), MGB (minor groove binder) or PNA (peptide nucleic acid) or any combination thereof.

15. A method according to claim 12, wherein nucleic acid amplification is detected by hybridization with a nucleic acid probe.

16. A method according to claim 12, wherein the probe used for nucleic acid amplification comprises DNA, PTO (phosphorothioate oligonucleotide), 2'-MOE (2'-methoxyethyl) oligonucleotide, RNA, LNA (locked nucleic acid), MGB (minor groove binder) or PNA (peptide nucleic acid), fluorescent labels or radiolabels or any combination thereof.

17. A method according to claim 12, wherein nucleic acid amplification is detected with a fluorescent dye.

18. A method according to claim 1, wherein nucleic acid, synthesized as a direct or indirect result of extension of the capture probe and/or the sequence of interest, is captured at a solid surface.

19. A capture probe for use in a method of qualitative and quantitative detecting a short nucleic acid sequence of interest, comprising a portion complementary to part of the sequence of interest and so capable of hybridizing thereto, and a portion non-complementary to the sequence of interest.

20. A capture probe according to claim 19, for use in a method according to claim 1.

21. A forward (upstream-, 5'-) primer for use in a method of qualitative and quantitative detecting a short nucleic acid sequence of interest, comprising a portion complementary to the extension of the capture probe and so capable of hybridizing thereto.

22. A forward primer according to claim 21, for use in a method according to claim 1.

23. A reverse (downstream-, 3'-) primer for use in a method of qualitative and quantitative detecting a short nucleic acid sequence of interest, comprising a portion complementary to the extension of the sequence of interest and so capable of hybridizing thereto.

24. A reverse primer according to claim 23, for use in a method according to claim 1.

25. A DNA probe for use in a method of qualitative and quantitative detecting a short nucleic acid sequence of interest, comprising a portion complementary to part of the sequence of interest and a portion complementary to the extension of the sequence of interest and so capable of hybridizing thereto.

26. A DNA probe according to claim 25, for use in a method according to claim 1.

27. A kit for use in qualitative and quantitative detecting the presence of a short nucleic acid sequence of interest in a sample, the kit comprising a capture probe in accordance with claim 19, and appropriate packaging means.

28. A kit according to claims 20, 22, 24, and 26, further comprising instructions for use in performing the method of claim 1.

29. A kit according to claims 20, 22, 24, and 26, further comprising one or more of the following: a nucleic acid polymerase; ribo- or deoxyribo-nucleotide triphosphates (labelled or unlabelled); labelling reagents; detection reagents; primers (labelled or unlabelled); probes (labelled or unlabelled); buffers.

30. Use of a method claimed in claim 1 for the detection of short nucleic acid sequences of about 8 -50 nucleotides in length as defined in claim 29.
